(19) **Europäisches Patentamt / European Patent Office / Office européen des brevets**

(11) **EP 4 382 544 A1**

(12) # EUROPEAN PATENT APPLICATION
published in accordance with Art. 153(4) EPC

(43) Date of publication:
**12.06.2024 Bulletin 2024/24**

(21) Application number: **22851503.7**

(22) Date of filing: **05.08.2022**

(51) International Patent Classification (IPC):
**C08F 251/00** (2006.01)    **C08F 220/06** (2006.01)
**C08F 222/02** (2006.01)    **C08F 222/06** (2006.01)
**C08F 2/16** (2006.01)

(52) Cooperative Patent Classification (CPC):
**A61K 8/25; A61K 8/37; C08B 31/00; C08B 31/06;
C08B 31/16; C08F 2/16; C08F 220/06;
C08F 222/02; C08F 222/06; C08F 251/00**

(86) International application number:
**PCT/BR2022/050311**

(87) International publication number:
**WO 2023/010197 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.08.2021 BR 102021015573**

(71) Applicant: **Chemyunion Ltda.**
**18087-101 Sorocaba (BR)**

(72) Inventors:
• **PRINCIVAL, Cleverson Rogério**
**18016-085 Sorocaba (BR)**

• **MUSSI, Lilian**
**18013-240 Sorocaba (BR)**
• **KATEKAWA, Edson**
**18030-160 Sorocaba (BR)**
• **MAGALHÃES, Wagner Vidal**
**18090-360 Sorocaba (BR)**
• **TOLEDO, Antônio Matias Navarrete De**
**18085-847 Sorocaba (BR)**

(74) Representative: **Grünecker Patent- und
Rechtsanwälte
PartG mbB
Leopoldstraße 4
80802 München (DE)**

(54) **PROCESS FOR SYNTHESISING A POLYMER, POLYMER, PROCESS FOR PRODUCING EMULSIFYING BASES, EMULSIFYING BASES, AND USE OF A POLYMER**

(57) The present invention comprises polysaccharide polymers modified with $\alpha$, $\beta$-unsaturated organic acids, production processes for said polymers and uses thereof. Furthermore, the present invention discloses emulsifying bases and their production processes. More specifically, the present invention discloses polymers produced by tetrasaccharides containing residues of $\alpha$-L-rhamnose, $\beta$-D-glucuronic acid and $\beta$-D-glucose modified with itaconic acid by graft polymerization processes and the use of said polymers as alternatives sustainable compared to polyacrylics commonly used in the cosmetics, pharmacy, veterinary and home care industries. The present invention refers to the fields of Personal Hygiene, Cosmetics, Chemistry, Biochemistry and Pharmaceuticals.

Figure 20

## Description

### Field of the Invention

The invention deals with polymer synthesis processes, polysaccharide polymers modified with $\alpha$, $\beta$-unsaturated organic acids, use of said polymers, emulsifying bases and their production processes. The present invention refers to the fields of Personal Hygiene, Cosmetics, Chemistry, Biochemistry, Veterinary and Pharmaceuticals.

### Background of the Invention

Synthetic polymers are part of our daily lives and represent one of the most versatile classes of materials that exist, presenting countless applications. In the cosmetic sector, acrylates occupy a prominent position. Among acrylates, we can mention sodium polyacrylate, a polymer derived from sodium salt and polyacrylic acid, which has numerous applications in the cosmetic area; superabsorbent polyacrylates (SAPs), which act as viscosity donors, emulsifiers and stabilizers, are of particular relevance. In addition to their use in cosmetics, superabsorbent polymers are used in candles, bandages, hot and cold therapy packs, diapers, and applications in agricultural business to absorb water in the soil, among other applications.

Environmental and sustainability issues are becoming critical for consumer and animal products companies, who must be aware of the environmental impact of their products throughout their life cycle. For example, the association of so-called microplastics (solid and spherical particles having less than 5 mm in size) with damage to the aquatic ecosystem has been reported. Accordingly, there is a growing concern about the global impact of polymers made up of polyacrylics, due to their non-renewable origin and their very slow degradation in nature, despite being the main responsible for positive aspects in texture and sensory properties in cosmetics formulations. An important behavior on the part of consumers of cosmetic products that deserves to be stood out is that they immediately evaluate the sensorial characteristics of a product when applied to the skin - and the perception gained in this process becomes one of the main motivations of purchase (and repurchase) process. For this reason, the search for natural and/or biodegradable alternatives to polyacrylates, without losing the sensorial characteristics and aspects of functional stability promoted by this class of polymers, is not only desirable, but also imperative.

The cosmetic, food and pharmaceutical industries have been investing in the use of some types of polysaccharides, such as gums and starches, as structuring and viscosity-increasing agents. Polysaccharides are complex carbohydrates that have many hydroxyl groups in their chemical structure, and in some cases, carboxyl and amide groups; these functional groups can interact strongly with water molecules, increasing the viscosity of a system. From a sensorial point of view, most polysaccharides have different properties from acrylates, since they promote low spreadability and high adhesiveness, properties not appreciated in cosmetic formulations for the skin.

Recently, the chemical and cosmetic industries have been looking for new alternatives to produce more sustainable, biodegradable, environmentally attractive polymers with appropriate sensorial properties for the cosmetic area.

Thus, there is a need for new products in the prior art - such as thickeners in cosmetic applications -, developed from renewable feedstocks and through sustainable synthetic processes, and which present themselves as an efficient alternative to the use of polyacrylates.

Therefore, from what can be inferred from the researched literature, no documents were found anticipating or suggesting teachings of the present invention, so that the solution proposed herein has novelty and inventive step compared to the prior art.

### Summary of the Invention

Thus, the present invention solves the problems of the prior art through the development of polysaccharide polymers modified with $\alpha$, $\beta$-unsaturated organic acids, from 3 to 5 carbons, production processes for said polymers and uses thereof. Furthermore, the present invention discloses emulsifying bases comprising said polymer and its production processes. More specifically, the present invention discloses polymers that are sustainable alternatives to polyacrylics commonly used in the cosmetics, pharmaceutical, veterinary and home care industries.

In a first object, the present invention discloses a polymer synthesis process comprising the following steps:

i) heating an aqueous reaction medium comprising from 0.5 mol/L to 2.0 mol/L of neutralizing agent and from 10% to 70% by mass of $\alpha$, $\beta$-unsaturated organic acids of 3 to 5 carbons to a temperature between 50 °C to 85 °C;

ii) adding polysaccharides to the system obtained at the end of step i), wherein the mass ratio of $\alpha$, $\beta$-unsaturated organic acids: polysaccharides is 5:95 to 80:20;

iii) removing gases in solution from the system obtained after carrying out step ii);

iv) adding to the system, as obtained at the end of step iii), a radical initiator in an amount of 0.001% to 10% by mass and, with the system in an inert atmosphere, rest it for 3h to 12h;

In a second object, the present invention discloses a polymer that is a polysaccharide modified with α, β-unsaturated organic acids of 3 to 5 carbons, wherein the mass ratio of α, β-unsaturated organic acids: polysaccharides is 5:95 to 80:20.

In a third object, the present invention discloses a process for producing emulsifying bases comprising the following steps:

i) adding to a system under nitrogen flow, stirring at 500rpm to 2000rpm and at a temperature of 50°C to 90°C:

a) 10% to 80% by mass of a polymer as previously defined;
b) 1% to 50% high and low HLB surfactants;
c) 2% to 50% waxes;
d) 5% to 60% vegetables oils;
e) 0.1% to 30% esters;
f) 0.1% to 30% polyols;

ii) promoting system flocking after solubilization of the system components obtained at the end of step i);
iii) isolating the product of interest obtained at the end of step ii).

In a fourth object, the present invention discloses an emulsifying base comprising in its composition from 10% to 80% by weight of a polymer as previously defined.

In a fifth object, the present invention discloses the use of a polymer, as previously defined, as a thickener, sensory modifier or film former.

In a sixth object, the present invention discloses the combination of the previously defined polymer with gums in cosmetic bases.

These and other objects of the invention will be immediately appreciated by those skilled in the art and will be described in detail below.

## Brief Description of the Figures.

The following figures are presented:

Figure 1 displays an FTIR-ATR infrared spectrum of the ISG1 copolymer.
Figure 2 displays a rheogram for IGM polymeric solution (1.5% (w/w)).
Figure 3 displays a rheogram for IGS1 polymer solution (1.5% (w/w)).
Figure 4 displays a rheogram for IGS2 polymer solution (1.5% (w/w)).
Figure 5 displays a rheogram for reference polymer solution (1.5% (w/w)).
Figure 6 displays a graph of viscosity ($\eta$) (Pa.s) as a function of strain rate for IGM polymeric solution (1.5% (w/w)).
Figure 7 displays a graph of viscosity ($\eta$) (Pa.s) as a function of the strain rate for IGS1 polymeric solution (1.5% (w/w)).
Figure 8 displays a graph of viscosity ($\eta$) (Pa.s) as a function of the strain rate for IGS2 polymeric solution (1.5% (w/w).
Figure 9 displays a graph of viscosity ($\eta$) (Pa.s) as a function of the strain rate for the reference polymeric solution (1.5% (w/w)).
Figure 10 displays a rheogram for the cream gel containing the reference polymer.
Figure 11 displays a rheogram for the cream gel containing the ISG1 polymer.
Figure 12 displays a graph of viscosity ($\eta$) (Pa.s) as a function of the strain rate $\gamma$ (s1) for the cream gel containing the reference polymer.
Figure 13 displays a graph of viscosity ($\eta$) (Pa.s) as a function of the strain rate $\gamma$ (s-1) for the cream gel containing the ISG1 polymer.
Figure 14 displays an optical microscopy of the cream gel (3% w/w) containing the IGS1 polymer.

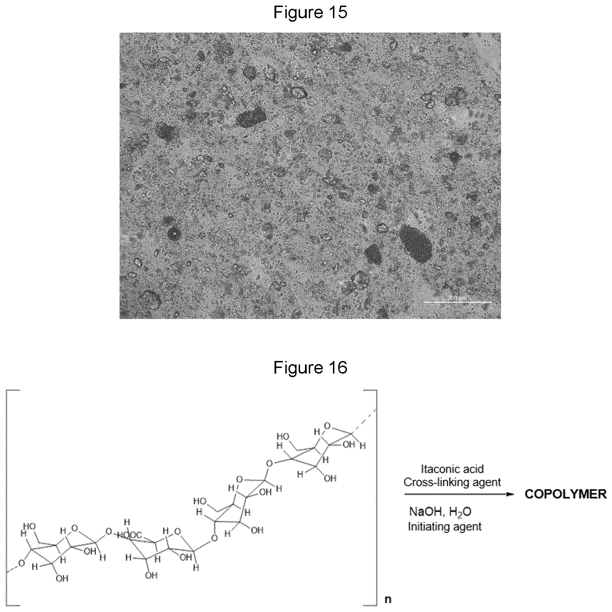

Figure 15 displays a polarized light microscopy of the cream gel (3% w/w) containing the IGS1 polymer.
Figure 16 displays a synthetic scheme of the graft polymerization reaction between polysaccharides and sodium itaconate.
Figure 17 displays a rheogram for the cosmetic base containing the IGS2 polymer (0.75% (w/w)) and xanthan.
Figure 18 displays the rheogram relating the viscosity as a function of the strain rate for a cosmetic base containing the IGS2 polymer (0.75% (w/w)) and xanthan gum.
Figure 19 displays the elastic modulus (G') and viscous modulus (G") as a function of amplitude for a cosmetic base containing the IGS2 polymer (0.75% (w/w)) and xanthan gum.

Figure 20 displays the Frequency Sweep behavior for a cosmetic base containing the IGS2 polymer (0.75% (w/w)) and xanthan gum.

Figure 21 displays polarized light microscopy for a cosmetic base containing the IGS2 polymer and xanthan gum at a 20x magnification.

## Detailed Description of the Invention

The present invention comprises polysaccharide polymers modified with $\alpha$, $\beta$-unsaturated organic acids, of 3 to 5 carbons, production processes of said polymers and uses thereof. Furthermore, the present invention discloses emulsifying bases comprising said polymers and their production processes. More specifically, the present invention discloses polymers produced by tetrasaccharides containing residues of $\alpha$-L-rhamnose, $\beta$-D-glucuronic acid and $\beta$-D-glucose or $\beta$-D-mannose modified with itaconic acid by graft polymerization processes and the use of said copolymers as sustainable alternatives to polyacrylics commonly used in the cosmetics, pharmacy, veterinary and home care industries.

The hybridization of natural polymers with synthetic polymers is of great interest in the development of new materials, as they can have superior properties in terms of rheology, viscosity and biodegradability compared to natural or synthetic polymers alone.

The present copolymer was developed through graft polymerization reactions analyzing the molecular structure of the monomers, intermolecular interactions, copolymers rheological properties as well as the origin of the feedstocks.

In one embodiment, $\alpha$,$\beta$-unsaturated organic acid, preferably itaconic acid, was used; polysaccharides, preferably tetrasaccharides containing residues of $\alpha$-L-rhamnose, $\beta$-D-glucuronic acid and $\beta$-D-glucose and/or $\beta$-D-mannose; ammonium persulfate as a radical initiator; sodium hydroxide as a neutralizing agent; pentaerythritol tetraallyl ether or N',N-methylene-bis-acrylamide as cross-linking agent; alkyl polyglycerol as a surfactant; and unsaturated vegetable oils comprising between $C_{12}$-$C_{22}$ to synthesize itaconic copolymers. The reactions were conducted in a temperature range between 40 C and 85 C.

Thus, the present invention presents an alternative to polyacrylics commonly used in industry, with satisfactory physical and chemical characteristics close to sodium polyacrylate and surprising performance in the intended applications. More specifically, polymers of the present patent application can be utilized in emulsifying bases, with low stickiness and high viscosity, promoting a higher and pleasant sensation to touch, a highly valuable property for acceptance, use and marketing of the product.

In a first object, the present invention discloses a polymer synthesis process comprising the following steps:

i) heating an aqueous reaction medium comprising from 0.5 mol/L to 2.0 mol/L of neutralizing agent and from 10% to 70% by mass of $\alpha$, $\beta$-unsaturated organic acids of 3 to 5 carbons to a temperature between 50 °C to 85 °C;

ii) adding polysaccharides to the system obtained at the end of step i), wherein the mass ratio of $\alpha$, $\beta$-unsaturated organic acids: polysaccharides is 5:95 to 80:20;

iii) adding a radical initiator in an amount of 0.001% to 10% by mass to the system as obtained at the end of step ii), and rest it for 3h to 12h;

In one embodiment, after step ii) the process comprises a step of removing gases in solution from the system obtained after carrying out step ii). In one embodiment, the system of step iii) is under an inert atmosphere.

In one embodiment, during step iii) it must rest from 4h to 12h. In one embodiment, during step iii) it must rest from 5h to 12h. In one embodiment, during step iii) it must rest from 5h to 10h.

In one embodiment, it is added from 0.001% to 5% by mass of a radical initiator. In one embodiment, it is added from 0.001% to 2% by mass of a radical initiator. In one embodiment, it is added from 0.10% to 5% by mass of a radical initiator. In one embodiment, it is added from 0.25% to 5% by mass of a radical initiator. In one embodiment, it is added 1% to 5% by mass of a radical initiator. In one embodiment, it is added 1% to 2% by mass of a radical initiator is added.

In one embodiment, the mass ratio of $\alpha$, $\beta$-unsaturated organic acids:polysaccharides is 20:80 to 80:20. In one embodiment, the mass ratio of $\alpha$, $\beta$-unsaturated organic acids: polysaccharides is 30:70 to 70:30. In one embodiment, the mass ratio of $\alpha$, $\beta$-unsaturated organic acids: polysaccharides is 40:60 to 60:40. In one embodiment, the mass ratio of $\alpha$, $\beta$-unsaturated organic acids: polysaccharides is 50:50.

In one embodiment, the temperature is 60°C to 75°C.

In one embodiment, the reaction medium comprises from 30% to 70% by mass of $\alpha$, $\beta$-unsaturated organic acids of 3 to 5 carbons. In one embodiment, the reaction medium comprises from 40% to 70% by mass of $\alpha$, $\beta$-unsaturated organic acids of 3 to 5 carbons. In one embodiment, the reaction medium comprises 50% to 70% by mass of $\alpha$, $\beta$-unsaturated organic acids of 3 to 5 carbons. In one embodiment, the reaction medium comprises 50% to 70% by mass of $\alpha$, $\beta$-unsaturated organic acids of 3 to 5 carbons. In one embodiment, the reaction medium comprises 40% to 60% by mass of $\alpha$, $\beta$-unsaturated organic acids of 3 to 5 carbons. In one embodiment, the reaction medium comprises 30% to 50% by mass of α, β-unsaturated organic acids of 3 to 5 carbons. In one embodiment, the reaction medium comprises 40% to 50% by mass of α, β-unsaturated organic acids of 3 to 5 carbons.

In one embodiment, the 3- to 5-carbon α,β-unsaturated organic acid is selected from the group consisting of maleic acid and its derivatives, fumaric acid and its derivatives, α-methylene-γ-butyrolactone, vinylpyrrolidone, acrylic acid, itaconic anhydride, maleic anhydride, methacryloyl oxyethyl trimethyl ammonium chloride and combinations thereof. In one embodiment, the 3- to 5-carbon α, β-unsaturated organic acid is itaconic acid.

In one embodiment, the polysaccharides are polysaccharides comprising residues of α-L-rhamnose, β-D-glucuronic acid, β-D-glucose, amylose, amylopectin, glucose, N-acetylglucosamine and glucosamine. In one embodiment, the polysaccharides are tetrasaccharides containing residues of α-L-rhamnose, β-D-glucuronic acid, β-D-glucose, β-D-mannose and α-galactopyranosides.

In one embodiment, the radical initiator is selected from the group consisting of ammonium persulfate, potassium persulfate, laccase enzyme, hydrogen peroxide, tert-butyl hydroperoxide, azobis (isobutyronitrile), benzoyl peroxide and combinations thereof. In one embodiment, the radical initiator is ammonium persulfate.

In one embodiment, the reaction medium comprises from 0.8 mol/L to 1.5mol/L of neutralizing agent. In one embodiment, the neutralizing agent is selected from the group consisting of sodium hydroxide, potassium hydroxide or ammonium carbonate.

In one embodiment, the reaction medium, as described in step i) additionally comprises from 1 % to 70% by mass of at least one of the components selected from cross-linking agents; surfactant agents and unsaturated vegetable oils comprising between $C_{12}$-$C_{22}$.

In one embodiment, the reaction medium comprises from 20% to 70% by mass of at least one of the components selected from cross-linking agents; surfactant agents and unsaturated vegetable oils comprising between $C_{12}$-$C_{22}$. In one embodiment, the reaction medium comprises from 30% to 70% by mass of at least one of the components selected from cross-linking agents; surfactant agents and unsaturated vegetable oils comprising between $C_{12}$-$C_{22}$. In one embodiment, the reaction medium comprises from 40% to 70% by mass of at least one of the components selected from cross-linking agents; surfactant agents and unsaturated vegetable oils comprised between $C_{12}$-$C_{22}$. In one embodiment, the reaction medium comprises from 20% to 50% by mass of at least one of the components selected from cross-linking agents; surfactant agents and unsaturated vegetable oils comrpising between $C_{12}$-$C_{22}$. In one embodiment, the reaction medium comprises from 30% to 60% by mass of at least one of the components selected from cross-linking agents; surfactant agents and unsaturated vegetable oils comprising between $C_{12}$-$C_{22}$. In one embodiment, the reaction medium comprises from 30% to 50% by mass of at least one of the components selected from cross-linking agents; surfactant agents and unsaturated vegetable oils comprising between $C_{12}$-$C_{22}$. In one embodiment, the reaction medium comprises 40% to 50% by mass of at least one of the components selected from cross-linking agents; surfactant agents and unsaturated vegetable oils comprising between $C_{12}$-$C_{22}$.

In one embodiment, the cross-linking agent is pentaerythritol tetraallyl ether or N',N-methylene-bis-acrylamide and the surfactant is alkyl polyglycerol.

In one embodiment, the process comprises adding 1% to 20% silicates. In one embodiment, 5% to 10% silicates are added. In one embodiment, the silicates are added in step i). In one embodiment, the silicates comprise kaolinites.

Silicon-based compounds have different sensorial and rheological properties and provide a soft and non-abrasive sensation. Furthermore, such compounds act as rheological modifiers, even at low concentrations, increasing the thixotropy of cosmetic formulations that lack this property, facilitating application and spreadability.

In a second object, the present invention discloses a polymer that is a polysaccharide modified with α, β-unsaturated organic acids of 3 to 5 carbons, wherein the mass ratio of α, β-unsaturated organic acids: polysaccharides is 5:95 at 80:20.

In one embodiment, the mass ratio of α, β-unsaturated organic acids:polysaccharides is 20:80 to 80:20. In one embodiment, the mass ratio of α, β-unsaturated organic acids:polysaccharides is 30:70 to 70:30. In one embodiment, the mass ratio of α, β-unsaturated organic acids: polysaccharides is 40:60 to 60:40. In one embodiment, the mass ratio of α, β-unsaturated organic acids:polysaccharides is 50:50.

In one embodiment, the polymer is additionally modified by at least one of the compounds selected from cross-linking agents; surfactants and unsaturated vegetable oils comprising between $C_{12}$-$C_{22}$.

In one embodiment, the polymer is produced by a process as previously defined.

In a third object, the present invention discloses a process for producing emulsifying bases comprising the following steps:

i) addition to a system under stirring from 500rpm to 2000rpm and at a temperature of 50°C to 90°C of:

a) 10% to 80% by mass of a polymer as previously defined;
b) 20% to 99% of at least one excipient;

ii) promoting system flocking after solubilization of the system components obtained at the end of step i);
iii) isolating the product of interest obtained at the end of step ii).

In one embodiment, the process system is under nitrogen flow.

In one embodiment, from 1% to 3% by mass of a polymer as previously defined is added. In one embodiment, from 1% to 5% of a polymer as previously defined is added. In one embodiment, from 10% to 70% by mass of a polymer as previously defined above is. In one embodiment, from 10% to 60% by mass of a polymer as previously defined above is. In one embodiment, from 10% to 50% by mass of a polymer as previously defined above is. In one embodiment, from 20% to 70% by mass of a polymer as previously defined is added. In one embodiment, from 30% to 70% by mass of a polymer as previously defined is added. In one embodiment, from 40% to 70% by mass of a polymer as previously defined is added. In one embodiment, from 40% to 60% by mass of a polymer as previously defined is added.

In one embodiment, the excipients are selected among high and low HLB surfactants, waxes, vegetable oils, esters and polyols. In one embodiment, in step i).from 1% to 50% high and low HLB surfactants, from 2% to 50% waxes, from 5% to 60% vegetable oils, 0.1% to 30% esters, and from 0.1% to 30% de polyols;are added

In one embodiment, in step i) from 50-90% water, preferably 70%, from 20% to 45% itaconic acid, preferably 25%, from 10% to 20% sodium hydroxide, preferably 13%, from 0.005% to 0.1% ammonium persulfate, preferably 0.01%, from 35% to 85% polysaccharide 35-85%, preferably 70%.

In one embodiment, in step i) from 30% to 50% water, from 1% to 20% itaconic acid, 1 - 20%, preferably 7%, from 1% to 20% sodium hydroxide, preferably 5%, from 0.005% to 0.1% ammonium persulfate, preferably 0.01%, from 10% to 30% polysaccharide, preferably 18%, from 3% to 10% surfactants, preferably 5.5%, and from 1% to 5% vegetable oils, preferably 2% are added.

In one embodiment, in step i) 30% to 50% water, 1% to 20% itaconic acid, preferably 7%, from 1% to 20% sodium hydroxide, preferably 5%, from 0.005% to 0.1% ammonium persulfate, preferably 0.01%, from 10% to 30% polysaccharide, preferably 18%, from 3% to 10% surfactants, preferably 5.5%, from 1% to 5% vegetable oils, preferably 2%, from 0.005 to 1% cross-linking agent, preferably 0.1% are added.

In one embodiment, the vegetable oils comprise sunflower oil. In one embodiment, the wax is candelilla wax and/or the surfactants comprise glyceryl monostearate and/or alkyl polyglucoside.

In a fourth object, the present invention discloses an emulsifying base comprising in its composition from 1% to 80% by mass of a polymer as previously defined.

In one embodiment, the emulsifying base comprises from 1% to 3% by mass of a polymer as previously defined. In one embodiment, the emulsifying base comprises from 1% to 80% by mass of a polymer as previously defined. In one embodiment, the emulsifying base comprises from 5% to 80% by mass of a polymer as previously defined. In one embodiment, the emulsifying base comprises from 3% to 80% by mass of a polymer as previously defined. In one embodiment, the emulsifying base comprises from 10% to 80% by mass of a polymer as previously defined. In one embodiment, the emulsifying base comprises from 10% to 70% by mass of a polymer as previously defined. In one embodiment, the emulsifying base comprises from 10% to 60% by mass of a polymer as previously defined. In one embodiment, the emulsifying base comprises from 10% to 50% by mass of a polymer as previously defined. In one embodiment, the emulsifying base comprises from 20% to 70% by mass of a polymer as previously defined. In one embodiment, the emulsifying base comprises from 30% to 70% by mass of a polymer as previously defined. In one embodiment, the emulsifying base comprises from 40% to 70% by mass of a polymer as previously defined. In one embodiment, the emulsifying base comprises from 40% to 60% by mass of a polymer as previously defined.

In one embodiment, the emulsifying base is produced by a process as previously defined.

In one embodiment, the emulsifying base presents behavior index below than 1. In one embodiment, the emulsifying base presents complex viscosity from 200 to 2500. In one embodiment, the emulsifying base presents apparent viscosity from 10 to 35 Pa.s. In one embodiment, the cosmetic base present consistency index from 3 to 50 Pa.sn. In one embodiment, the cosmetic base present consistency index from 4 to 45 Pa.sn.

In a fifth object, the present invention discloses the use of a polymer as previously defined as a thickener, sensory modifier or film former.

In one embodiment, the polymer used is comprised of an emulsifying base as previously defined.

In one embodiment, the polymer is used in cosmetic, pharmaceutical, veterinary and cleaning product applications.

In the present invention, the following is understood as:

High and low HLB surfactants: as used herein, the term "High and low HLB surfactants" stands for hydrophilic-lipophilic balance and refers to any amphipathic organic compounds that present in its molecule a polar part and a non-polar part.
Flocking: as used here, the term "Flocking" refers to the process of rapid cooling of the system (thermal shock), leading to the structuring of the system. Thus, the aggregation of particles occurs, the so-called "flakes" or "floccules".

Emulsifying base: as used herein, the term "Emulsifying base" refers to gels and bases capable of emulsifying and changing the rheology of compositions.

Stickiness: as used herein, the term "stickiness" can be understood as degree in which the hand surface adheres to skin, considering that the measurement method would be to strongly press the hand above the area where the test material (cosmetic cream, for example) was applied and remove it, repeating this process to evaluate stickiness in a subjective scale of 0 (soft) abd 9 (hard). Stickiness is a common term in the prior art and cited by diverse skilled in the art (Dubuisson (2018), Gilbert (2012), Blok (2021) e Kusakari (2003)), being possible to calculate the stickiness from these diverse parameters.

In one embodiment of the present patent application, the organic acid is itaconic acid, a biotechnological source monomer and highly biodegradable. Surprisingly this molecule showed changes in sensorial profile of emulsifying bases, providing enhancing sensorial aspect and increase in biodegradability of polymeric materials. In addition, as being a dicarboxylic monomer, it was obtained improved properties compared to other copolymers, such as enhance water absorption, rheological profile and high rate of biodegradability

In one embodiment, the tetrasaccharides herein described presented improved reactional performance, facilitating reaction scale-up. Another important factor is related to the sensorial profile, provided by the polymer formed by tetrasaccharides with sodium itaconate. The graft polymers presented high solubility in water, a highly appreciated property in cosmetic formulations, as well as a high rate o biodegradability.

Thus, modifying gums with polysaccharides and organic dicarboxylic acids promoted a stickiness reduction, a non-appreciated property in cosmetic formulations, enhancing the sensorial profile of products.

**Examples**

Example I - Production processes of the itaconic polysaccharide copolymer

Five examples of production processes for the itaconic polysaccharide copolymer were developed, namely:

Production example 1 - in a 2 Liter reactor coupled to a mechanical stirrer and under nitrogen flow, water (0.750 Liter), sodium hydroxide (0.7375 mol), itaconic acid (0.384 mol) were added. The reaction system was heated to 60-70°C and then the tetrasaccharide $\alpha$-L-rhamnose, $\beta$-D-glucuronic acid and $\beta$-D-glucose (162 g) were added and then vacuum (35 mbar) was applied to remove gases present in solution. In the next step, ammonium persulfate (2.2 mmol) was added as a radical initiator and the reaction was monitored for 12 hours under heating at a temperature of 60-75°C and nitrogen flow. Subsequently, the product was precipitated in ethanol and filtered in a Büchner funnel, to remove residual monomers and impurities, and dried in a vacuum oven at constant temperature, providing the product with 79-83% yield.

Production example 2 - in a 2 Liter reactor coupled to a mechanical stirrer and under nitrogen flow, water (1 Liter), sodium hydroxide (0.7375 mol), itaconic acid (0.4 mol) were added. The reaction system was heated to 60-70°C and then the tetrasaccharide $\alpha$-L-rhamnose, $\beta$-D-glucuronic acid and $\beta$-D-glucose (150 g) were added and then vacuum (35 mbar) was applied to remove gases present in solution. In the next step, ammonium persulfate (2.5 mmol) was added as a radical initiator and the reaction was monitored for 12 hours under heating at a temperature of 60-75°C and nitrogen flow. Subsequently, the product was precipitated in ethanol and filtered in a Büchner funnel, to remove residual monomers and impurities, and dried in a vacuum oven at constant temperature, providing the product with 85-91% yield.

Production example 3 - in a 2 Liter reactor coupled to a mechanical stirrer and under nitrogen flow, water (0.750 Liter), sodium hydroxide (1.4 mol), itaconic acid (0.768 mol) were added. The reaction system was heated to 60-70°C and then the tetrasaccharide $\alpha$-L-rhamnose, $\beta$-D-glucuronic acid and $\beta$-D-glucose (90 g) were added and then vacuum (35 mbar) was applied to remove gases present in solution. In the next step, ammonium persulfate (2.2 mmol) was added as a radical initiator and the reaction was monitored for 12 hours under heating at a temperature of 60-75°C and nitrogen flow. Subsequently, the product was precipitated in ethanol and filtered in a Büchner funnel, to remove residual monomers and impurities, and dried in a vacuum oven at constant temperature, providing the product with 85-89% yield.

Production example 4 - in a 2 Liter reactor coupled to a mechanical stirrer and under nitrogen flow, water (0.750 Liter), sodium hydroxide (0.7375 mol), itaconic acid (0.384 mol), N',N- were added methylene-bis-acrylamide as a cross-linking agent at a concentration of ($8.6 \times 10^{-4}$ mol/L), polyglyceryl10 decaoleate (2%), polyglyceryl10 laurate (2%) and sunflower oil (5%). The reaction system was heated to 60-70°C and then the tetrasaccharide $\alpha$-L-rhamnose, $\beta$-D-glucuronic acid and $\beta$-D-glucose (162 g) were added and then vacuum (35 mbar) was applied to remove gases present in solution. In the next step, ammonium persulfate (2.2 mmol) was added as a radical initiator and the reaction was monitored for 12 hours under heating at a temperature of 60-75°C and nitrogen flow. Subsequently, the product was precipitated in ethanol and filtered in a Büchner funnel, to remove residual monomers and impurities, and dried in a vacuum oven at constant temperature, providing the product with 88-91% yield.

Production example 5 - in a 2 Liter reactor coupled to a mechanical stirrer and under nitrogen flow, water (0.750 Liter), sodium hydroxide (0.7375 mol), itaconic acid (0.384 mol) and N',N- were added methylene-bis-acrylamide as a cross-linking agent at a concentration of ($8.6 \times 10^{-4}$ mol/L). The reaction system was heated to 60-70°C and then added the pentasaccharide containing units of glucose, mannose and glucuronic acid in a ratio of 2: 2: 1 (3 g) and tetrasaccharide α-L-rhamnose, β-D acid -glucuronic acid and β-D-glucose (50 g) and then vacuum (35 mbar) was applied to remove gases present in solution. In the next step, ammonium persulfate (2.2 mmol) was added as a radical initiator and the reaction was monitored for 12 hours under heating at a temperature of 60-75°C and nitrogen flow. Subsequently, the product was precipitated in ethanol and filtered in a Büchner funnel, to remove residual monomers and impurities, and dried in a vacuum oven at constant temperature, providing the product with 75-78% yield.

For nomenclature purposes, IGM refers to a polymeric composition for application as a sensory and viscosity modifier containing sodium polyitaconate in a concentration range of 0.1-20% and tetrasaccharide containing, preferably, α-L residues. -rhamnose, β-D-glucuronic acid and β-D-glucose and/or β-D-mannose in a concentration range of 80-99%.

IGS1 refers to the polymer produced according to the production examples that do not comprise cross-linking agents, likewise, IGS2 refers to the polymers produced according to the examples that comprise said agents and, additionally, unsaturated vegetable oils.

Example II - Production of the emulsifying base (organogel) containing the itaconic polysaccharide copolymer.

The self-emulsifying base was prepared by combining sunflower oil (30-45%), containing high oleic group content (HOSO) as organic fluid, which was structured with candelilla wax (2%), glyceryl monostearate (13 %) and xylitol sesquicaprylate (polyol esters) (1%) which were heated at 80°C under nitrogen flow until complete solubilization of the components. Subsequently, the itaconic copolymer α-L-rhamnose polysaccharide, β-D-glucuronic acid and β-D-glucose were slowly added under strong stirring (1000 rpm) in a concentration range of 35-50%. After stirring for 30 minutes, the content was flaked to form the self-emulsifying base.

Example III - Characterization of itaconic polysaccharide copolymers by FTIR-ATR.

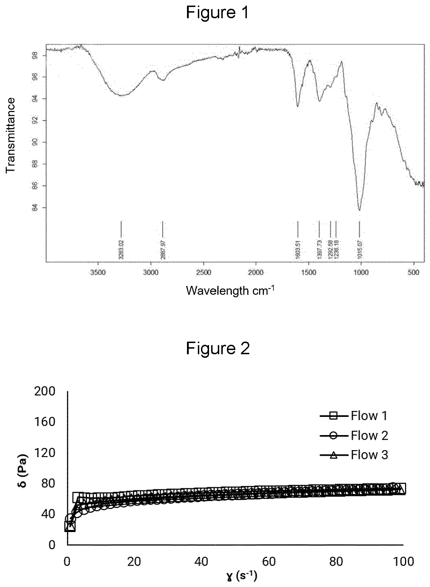

As a model for structural elucidation of copolymers, we observed in the FTIR-ATR infrared spectrum of the ISG1 copolymer, two bands at 3283 and 2887 $cm^{-1}$ referring to the stretching vibrations of -OH and aliphatic CH, respectively. Two other absorption bands at 1603 and 1397 $cm^{-1}$ refer to carboxylate groups. The band at 1603 $cm^{-1}$ is due to the asymmetric stretching of the $COO^{-}$ group, while the absorption band at 1397 $cm^{-1}$ can be attributed to the symmetric stretching of the $COO^{-}$. The broad wavelength signal is due to the slight difference in stretching frequency between the COO- monocarboxylates and dicarboxylates present in the copolymer composition and originating from the itaconic group. Furthermore, we can see, in Figure 1, a band at 1430 $cm^{-1}$ referring to the angular deformation of the CH2 group adjacent to the carbonyl and another stretching at 1015 $cm^{-1}$ referring to the C-O bond present in the copolymer, characterizing the formation of the ISG1 copolymer.

Example IV - Application of the emulsifying base (organogel).

In a 250 mL beaker, 3 to 10% of the emulsifying base and water (90 to 97%) were added, then the formulation was shaken (500 to 800 rpm) to form the cream gel or serum.

Example V - Rheological evaluation of itaconic polysaccharide copolymers

The copolymers (IGM, ISG1 and ISG2) were challenged in rheology studies and their rheological properties were analyzed and compared against the reference polymer (sodium polyacrylate), as the type of polymer used in the gel formulation can influence the rheological behavior this and, therefore, can influence the physical stability of the product, as well as its behavior on the skin, interfering with the release of the active ingredient by the vehicle and the formation of a film on the skin.

Example VI - Production of cosmetic base containing the IGS2 polymer and xanthan gum.

The cosmetic base, which can be applied for facial treatment, makeup base and/or body treatment, was prepared by combining castor oil (40 - 80%) as an organic fluid, which was structured with $C_{18}$-$C_{22}$ triglycerides (3.0 - 10%), IGS2 polymer (10 - 30%), cetostearyl alcohol (0.25 - 1.0%), alkyl poly-glucoside (15 - 30%), xanthan gum (1.0 - 3.0) and tocopherol as an antioxidant agent. The components were mixed and heated to 80°C and subjected to strong mechanical agitation for 30 minutes to completely homogenize the system. Subsequently, the cosmetic base was cooled and flaked.

The use concentrations of the different polymeric solutions were (1.5% (w/w)) in water. Furthermore, the emulsifying bases containing the copolymers were also analyzed and the results are described in this document.

An amount of sample necessary to cover the entire surface of the cone and plate system was placed on the plate, with the excess sample being carefully removed with a spatula.

The rheological properties of different polymeric solutions (1.5% (w/w)) as well as aqueous solutions of two emulsifying bases (3% w/w) were evaluated at 25°C using a hybrid rheometer (model HR2, Discovery Serie, TA Instruments, New Castle, USA). The geometry stipulated for the analyzes was the stainless-steel plate-plate type with 40 mm in diameter.

Thixotropy is a rheological property of fluids. This can be understood as a time-dependent decrease in viscosity when a tension is applied to the sample that was at rest and subsequent recovery of the initial viscosity when the tension is discontinued (MEWIS & WAGNER, 2009). Thixotropy can be verified in stress versus strain rate rheograms. Thixotropic materials have an area between the rising and falling curves, indicating that flow resistance is being reduced with shear time.

Steady flow curves were performed with strain rate varying between 0 and 100 $s_{-1}$ in three sweeps (ascending, descending and ascending, respectively). The curves were named Flow 1, 2 and 3 respectively). Using TRIOS software calculations, it was possible to determine the thixotropy of the materials. The data obtained by the third curve were transformed into viscosity data as a function of the strain rate and adjusted to the Power Law fluid mathematical model, which is described by equation 1:

**Equation 1** - Power Law

$$\eta = K . \dot{\gamma}^{n-1} \ (1)$$

wherein $\eta$ = apparent viscosity (Pa.s); K = consistency index (Pa.sn); $\gamma^{\cdot}$ = strain rate (s-1) and n = fluid behavior index. Thixotropy values were calculated using the TRIOS v software. 5.0 (TA Instruments, New Castle, USA). Adjustments to the mathematical model were made in the Microsoft Excel 2013 program.

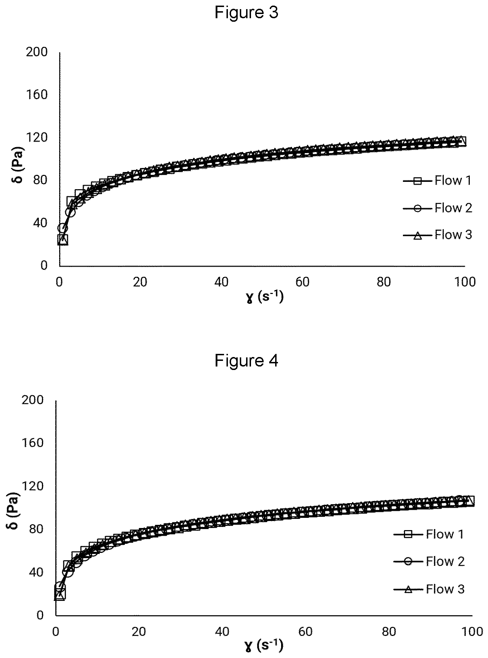

Figures 2 to 5 show the rheograms of polymeric solutions (IGM, IGS1, IGS2 and reference polymer) at a concentration of 1.5% (w/w). All solutions showed small thixotropy (normalized values, Table 1), calculated by the area between the Flow 1 and Flow 2 curves.

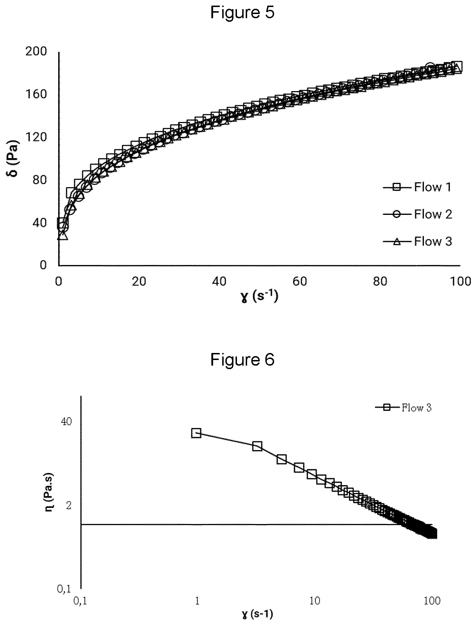

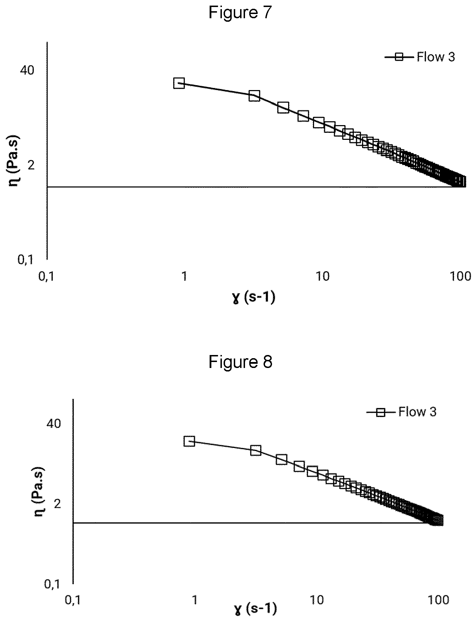

The graphs in Figures 6 to 9 illustrate the behavior of viscosity ($\eta$) (Pa.s) as a function of the strain rate. The model adjustment parameters and respective coefficients of determination ($R^2$) are illustrated in Table 1.

**Table 1** - Normalized thixotropy and other rheological parameters of different polymeric solutions (k, n and $R^2$ calculated by adjusting the Power Law model).

| Parameter | 1.5% polymeric solution (w/w) | | | |
|---|---|---|---|---|
| Polymer | IGM | IGS1 | IGS2 | Reference Polymer |
| Normalized thixotropy ($\times 10^{-4}$) ($s^{-1}$) | 0.31 | 0.53 | 1.3 | 2.5 |
| Consistency Index (k) (Pa.s$^n$) | 18.7 | 38.45 | 46.3 | 20.98 |
| Behavior Index (n) (-) | 0.71 | 0.74 | 0.77 | 0.62 |
| Determination coefficient ($R^2$) (-) | 0.99 | 0.99 | 0.99 | 0.99 |

All polymeric samples studied showed negligible values of normalized thixotropy. The sample with the lowest thixotropy value was the IGM polymeric solution ($0.31 \times 10^{-4} s^{-1}$). The synthesis processes led to the formation of the IGS1 and IGS2 polymers, these polymers presented rheological results close to those found for the reference polymer solutions, (Table 1).

All polymeric solutions showed good adjustment to the Power Law model, which was verified by the coefficients of determination obtained (Table 1). Fluids that fit this model are time-independent non-Newtonian fluids. The mathematical equation that represents them has two parameters that characterize them: the consistency index (k) and the behavior index (n). The behavior index n (-) characterizes the fluid in pseudoplastics or dilatants. For n < 1, the fluids are called pseudoplastic and are characterized by a reduction in viscosity with an increase in the deformation rate. For n > 1, fluids are called dilatant and are characterized by an increase in viscosity with an increase in the deformation rate (STEFFE, 1996). When n =1, the fluid is considered Newtonian. The consistency index k (Pa.s") is a constant dependent on the process temperature (STEFFE, 1996).

All polymeric solutions studied were characterized as pseudoplastic with behavior indices varying between 0.62 (reference polymer solution, highest plasticity) and 0.77 (IGS2 solution, lowest plasticity among the studied solutions). Polymeric solutions generally exhibit pseudoplasticity. It is believed that at higher deformation rates, the polymer chains align in the direction of flow, thus producing less resistance with a consequent decrease in the viscosity of the solution (SARAMITO, 2016).

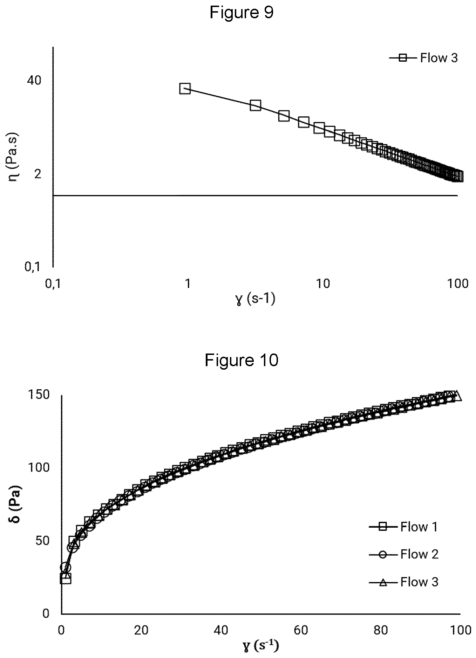

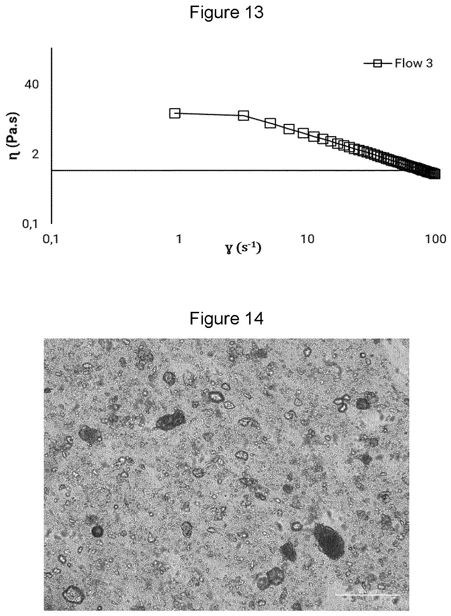

Polymers were challenged in cosmetic formulations for the preparation of emulsifying bases containing surfactants, emollients and vegetable oils. The emulsifying bases were dispersed in aqueous solutions to form a cream gel at a concentration of (3% w/w) and the results obtained are shown in Figures 10 and 13 and in table 2.

**Table 2** - Rheological parameters found for the cream gel containing the reference polymers and ISG1 (k, n and $R^2$ calculated by adjusting the Power Law model).

| Parameter | Cream gel containing polymer or reference 3% (w/w) | |
|---|---|---|
| Polymer | Formulated with reference | Formulated with IGS1 |
| Normalized thixotropy ($\times 10^{-4}$) ($s^{-1}$) | 4.3 | 4.9 |
| Consistency Index (k) (Pa.s) | 30.81 | 20.15 |
| Behavior Index (n) (-) | 0.66 | 0.67 |
| Determination coefficient ($R^2$) (-) | 0.99 | 0.98 |

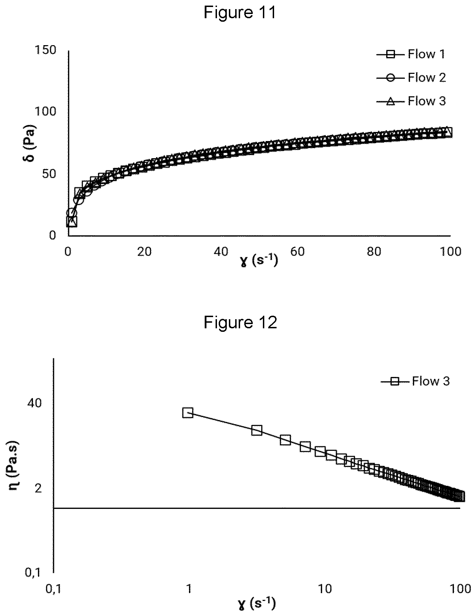

Figures 12 and 13 show the viscosity behavior as a function of the strain rate for the cream gel formulated with the reference polymers or ISG1. The results obtained in rheological studies of cream gels, containing reference polymer and ISG1, showed similar results in thixotropy and behavior index, differing only in viscosity values, in which the reference polymer presented slightly higher values.

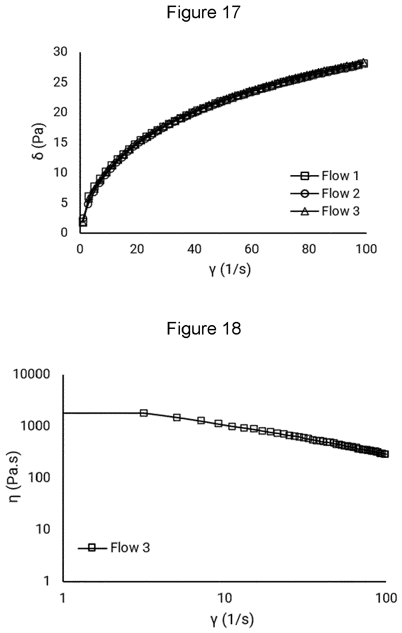

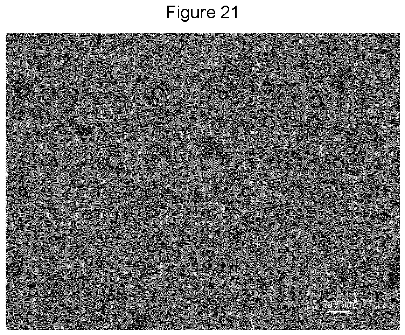

Another study carried out was the preparation of serum-type cosmetic bases containing xanthan gum and the IGS2 polymer in its formulation, which is characterized by being a base with a light texture, quick absorption and easy spreadability. The purpose of this study was to challenge the IGS2 polymer compared to gums recognized for having high adhesiveness values (stickiness, a term used in the cosmetic field) - a property not appreciated in cosmetic formulations. The IGS2 polymer was able to act as a sensory modifier, reducing stickiness, making the cosmetic base pleasant to the touch. Therefore, rheological studies were carried out on this cosmetic base and Figures 17 and 21 respectively illustrate the flow curve of the material (shear stress versus strain rate) and the viscosity behavior as a function of strain rate. The material flow curve was performed with three consecutive ramps (ascending, descending and ascending) varying the strain rate from 0 to 100 $s^{-1}$. From these data it was possible to obtain thixotropy results and subsequently model them to the existing fluid equations.

Analyzing Figure 17, it is possible to observe an overlap of the curves of the respective ramps. This fact shows that the material has low thixotropy. The data obtained by the third curve were adjusted to the Power Law fluid mathematical model, which is described by equation 1: Equation 1 - Power Law-. $\eta = K \cdot \dot{\gamma}^n$ wherein $\eta$ = apparent viscosity (Pa.s); K = consistency index (Pa.sn); $\dot{\gamma}$ = strain rate (s-1) and n = fluid behavior index.

The serum-type cosmetic base containing the ISG2 polymer and xanthan gum showed a good fit to the Power Law model, which was verified by the coefficient of determination obtained (Table 3). Fluids that fit this model are time-independent non-Newtonian fluids. The mathematical equation that represents them has two parameters that characterize them: the consistency index (k) and the behavior index (n). The behavior index n (-) characterizes the fluid in pseudoplastics or dilatants. For n < 1, the fluids are called pseudoplastic and are characterized by a reduction in viscosity with an increase in the deformation rate. For n > 1, fluids are called dilatant and are characterized by an increase in viscosity with an increase in the deformation rate (STEFFE, 1996). When n =1, the fluid is considered Newtonian. The consistency index k (Pa.sn) is a constant dependent on the process temperature (STEFFE, 1996). According to the behavior index obtained by the study and demonstrated in table 3 in Figure 18, the serum-type cosmetic base proved to be a pseudoplastic fluid.

Table 3. Cosmetic-based rheological parameters (k, n and $R^2$ calculated by adjusting the Power Law model).

| Parameters | Results |
|---|---|
| Consistency Index (k) (Pa.s$^n$) | 4.19 |
| Behavior Index (n) (-) | 0.42 |
| Determination coefficient ($R^2$) (-) | 0.99 |

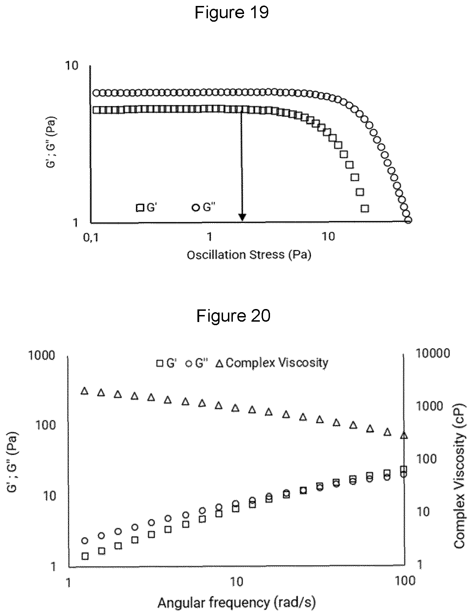

In contrast to flow curve analysis, which is a stationary test, amplitude sweep is an oscillatory test that uses an increasing amplitude to evaluate sample properties such as its rheological stability, Figure 19.

The sweep amplitude test is important for determining the linear region of the material - that is, the region in which the deformations are reversible. This obtained result is used in subsequent analyses, such as Frequency Sweep and Temperature Ramps.

Observing Figure 19, it is possible to observe G" > G'; Therefore, the cosmetic base studied is a viscoelastic fluid and the material will always be in fluid form. The limit of the linear region (Yield stress), observed by the graph, is around 2 Pa.

Figure 20 illustrates the Frequency Sweep results for the cosmetic base containing the IGS2 polymer and xanthan gum. For this test, a constant voltage of 1 Pa (linear region) was used. The Frequency Sweep test generates a rheological fingerprint of the material. Thus, we can evaluate the solid nature (G'), liquid nature (G") and complex viscosity of the material over a frequency range. This test is valuable for evaluating viscoelastic behavior and organization of polymers in different matrices.

It can be seen in Figure 20 that at low frequencies the material has a predominance of G" - that is, it is predominantly fluid. As the frequency is increased, G' begins to overlap G", showing that the material when subjected to forces in a short space of time will have relevant solid characteristics.

The serum was analyzed by polarized light microscopy. It was possible to observe the formation of a microstructure consisting of a continuous fluid and translucent phase, which contains two types of structures: oily drops and small fragments of oleogel, in a homogeneous way (Figure 21).

From the results obtained, it was possible to conclude that the serum proved to be a pseudoplastic fluid with low thixotropic values. Its linear region showed a greater predominance of the viscous modulus (G") in relation to the elastic modulus (G'), characterizing a fluid material. The frequency sweep results showed that the base appears fluid in short force application times and this behavior is modified to solid characteristics when this force application time is reduced. Polarized light microscopy tests showed that the cosmetic base is made up of a continuous and transparent matrix that carries oily droplets and oleogel fragments inside.

The examples shown here are intended only to exemplify one of the countless ways of carrying out the invention, however without limiting its scope.

Those skilled in the art will value the knowledge showed herein and will be able to reproduce the invention in the presented embodiments and in other variants and alternatives, covered by the scope of the following claims.

**Claims**

**1.** A process for synthesizing a polymer **characterized in that** it comprises the following steps:

   i) heating an aqueous reaction medium comprising from 0.5 mol/L to 2.0 mol/L of neutralizing agent and from 10% to 70% by mass of $\alpha$, $\beta$-unsaturated organic acids of 3 to 5 carbons at a temperature between 50 °C to 85 °C;
   ii) adding at least one polysaccharides to the system obtained at the end of step i), wherein the mass ratio of $\alpha$, $\beta$-unsaturated organic acids:polysaccharides is 5:95 to 80:20;
   iii) adding a radical initiator in an amount of 0.001% to 10% by mass to the system, as obtained at the end of step ii), and for 3h to 12h.

**2.** The process according to claim 1, **characterized in that** $\alpha$, $\beta$-unsaturated organic acid of 3 to 5 carbons is selected from the group consisting of maleic acid and its derivatives, fumaric acid and its derivatives, $\alpha$-methylene-$\gamma$-butyrolactone, vinylpyrrolidone, acrylic acid, itaconic anhydride, maleic anhydride, methacryloyl oxyethyl trimethyl ammonium chloride and combinations thereof.

**3.** The process according to claim 1 or 2, **characterized in that** the polysaccharides comprise, not exclusively, residues of $\alpha$-L-rhamnose, $\beta$-D-glucuronic acid, $\beta$-D-glucose, amylose, amylopectin, glucose, N-acetylglucosamine and glucosamine.

**4.** The process according to any of the preceding claims, **characterized in that** the radical initiator is selected from the group consisting of ammonium persulfate, potassium persulfate, laccase enzyme, hydrogen peroxide, tert-butyl hydroperoxide, azobis(isobutyronitrile), hydrogen peroxide benzoyl and combinations thereof.

**5.** The process according to any one of the preceding claims, **characterized in that** the neutralizing agent is sodium hydroxide, potassium hydroxide or ammonium carbonate.

**6.** The process according to any one of the preceding claims, **characterized in that** the reaction medium as described in step i) additionally comprises from 1% to 70% by mass of at least one of the components selected from cross-linking agents; surfactant agents and unsaturated vegetable oils comprising $C_{12}$-$C_{22}$.

7. The process according to claim 6, **characterized in that** the cross-linking agent is pentaerythritol tetraallyl ether or N',N-methylene-bis-acrylamide and the surfactant agent is alkyl polyglycerol.

8. A polymer **characterized in that** it is a polysaccharide modified with $\alpha$, $\beta$-unsaturated organic acids of 3 to 5 carbons, wherein the mass ratio of $\alpha$, $\beta$-unsaturated organic acids: polysaccharides is 5:95 to 80:20.

9. The polymer according to claim 8, **characterized in that** it is additionally modified by at least one of the compounds selected from cross-linking agents; surfactants and unsaturated vegetable oils comprising $C_{12}$-$C_{22}$.

10. The polymer according to claim 8 or 9, **characterized in that** it is produced by a process as defined in any one of claims 1 to 7.

11. A process for producing an emulsifying base **characterized in that** it comprises the following steps:

    i) addition to a system under stirring from 500rpm to 2000rpm and at a temperature of 50 °C to 90 °C of:

        a) 1% to 80% by mass of a polymer as defined in any one of claims 8 to 10;
        b) 20% to 99% of at least one excipient;

    ii) promoting system flocking after solubilization of the system components obtained at the end of step i);
    iii) isolating the product of interest obtained at the end of step ii).

12. An emulsifying base **characterized in that** it comprises in its composition from 10% to 80% by mass of a polymer as defined in any one of claims 1 to 7.

13. The emulsifying base, according to claim 12, **characterized in that** it is produced by a process as defined in claim 11.

14. Use of a polymer as defined in any one of claims 1 to 7 **characterized by** being as a thickener, sensory modifier or film former.

15. The use according to claim 14, **characterized in that** the polymer is present in an emulsifying base as defined in claim 12 or 13.

## Figure 1

## Figure 2

Figure 3

Figure 4

## Figure 5

## Figure 6

Figure 7

Figure 8

## Figure 9

## Figure 10

Figure 11

Figure 12

Figure 13

Figure 14

Figure 15

Figure 16

Figure 17

Figure 18

Figure 19

Figure 20

Figure 21

## INTERNATIONAL SEARCH REPORT

| | International application No. |
|---|---|
| | **PCT/BR2022/050311** |

| A. CLASSIFICATION OF SUBJECT MATTER |
|---|
| **IPC: C08F251/00 (2006.01), C08F220/06 (2006.01), C08F222/02 (2006.01), C08F222/06 (2006.01), C08F2/16 (2006.01)** |
| **CPC: C08F251/00, C08F220/06, C08F222/02, C08F222/06, C08F2/16** |
| According to International Patent Classification (IPC) or to both national classification and IPC |

| B. FIELDS SEARCHED |
|---|
| Minimum documentation searched (classification system followed by classification symbols) |
| **IPC: C08F251/00, C08F220/06, C08F222/02, C08F222/06, C08F2/16** |
| Documentation searched other than minimum documentation to the extent that such documents are included in the fields searched |
| **Banco de Patentes do INPI-BR** |
| Electronic data base consulted during the international search (name of data base and, where practicable, search terms used) |
| **Derwent Innovation, Espacenet, Google Patents** |

| C. DOCUMENTS CONSIDERED TO BE RELEVANT | | |
|---|---|---|
| Category* | Citation of document, with indication, where appropriate, of the relevant passages | Relevant to claim No. |
| X<br>Y | **US 5227446 A (BASF AG [DE])**<br>**13 July 1993 (1993-07-13)**<br>**See whole document.**<br><br>-------------------------------------------- | 1 to 10<br>11 to 15 |
| Y | **US 4029616 A (SUMITOMO CHEMICAL CO)**<br>**14 June 1977 (1977-06-14)**<br>**See whole document.**<br><br>-------------------------------------------- | 14 |
| Y | **US 8853144 B2 ( ECOLAB USA INC [US])**<br>**07 October 2014 (2014-10-07)**<br>**See whole document.**<br><br>-------------------------------------------- | 11 to 13, 15 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **30/09/2022** | **10/10/2022** |

| Name and mailing address of the  **ISA/BR** | Authorized officer |
|---|---|
| Facsimile No. | Telephone No. |

Form PCT/ISA/210 (second sheet) (January 2015)

INTERNATIONAL SEARCH REPORT
Information on patent family members

International application No.

**PCT/BR2022/050311**

| US 5227446 A | 1993-07-13 | AU 7017291 A | 1991-08-08 |
| | | AU 628712 B2 | 1992-09-17 |
| | | CA 2034943 A1 | 1991-08-04 |
| | | DE 4003172 A1 | 1991-08-08 |
| | | DE 59107176 D1 | 1996-02-15 |
| | | EP 0441197 A2 | 1991-08-14 |
| | | ES 2081377 T3 | 1996-03-01 |
| | | JP H04356513 A | 1992-12-10 |
| US 4029616 A | 1977-06-14 | CA 1049674 A | 1979-02-27 |
| | | DE 2504117 A1 | 1975-08-07 |
| | | FR 2259872 A1 | 1975-08-29 |
| | | GB 1496217 A | 1977-12-30 |
| | | JP S50108391 A | 1975-08-26 |
| | | JP S5411358 B2 | 1979-05-14 |
| US 8853144 B2 | 2014-10-07 | US 2013035275 A1 | 2013-02-07 |
| | | US 2013035274 A1 | 2013-02-07 |
| | | US 8636918 B2 | 2014-01-28 |
| | | US 2013035276 A1 | 2013-02-07 |
| | | US 8679366 B2 | 2014-03-25 |
| | | US 2013035277 A1 | 2013-02-07 |
| | | US 8841246 B2 | 2014-09-23 |
| | | US 2015005217 A1 | 2015-01-01 |
| | | US 9309489 B2 | 2016-04-12 |
| | | US 2015005218 A1 | 2015-01-01 |
| | | US 9309490 B2 | 2016-04-12 |
| | | US 2013035273 A1 | 2013-02-07 |
| | | WO 2013022746 A2 | 2013-02-14 |
| | | WO 2013022762 A1 | 2013-02-14 |
| | | WO 2013022769 A1 | 2013-02-14 |
| | | WO 2013022777 A1 | 2013-02-14 |
| | | WO 2013022781 A2 | 2013-02-14 |

Form PCT/ISA/210 (patent family annex) (January 2015)